# EUROPÄISCHE PATENTANMELDUNG

(11) **EP 4 385 542 A1**
(43) Veröffentlichungstag der Anmeldung: **19.06.2024**
(21) Anmeldenummer: 22213561.8
(22) Anmeldetag: 14.12.2022
(51) Int. Cl.: A61M 5/28, A61J 1/14, A61M 5/31, B65D 51/00, A61M 5/315

(54) **NADELKOPF FÜR EIN INJEKTIONSGERÄT FÜR EINEN MEDIZINISCHEN WIRKSTOFF UND INJEKTIONS-GERÄT MIT EINEM SOLCHEN NADELKOPF**

(71) Anmelder: Inductio AG, 4500 Solothurn (CH)
(72) Erfinder: KOLLER, Horst, 8730 Uznach (CH)
(74) Vertreter: Tergau & Walkenhorst Intellectual Property GmbH

(57) **Zusammenfassung**

Ein Nadelkopf (6, 6') für ein Injektionsgerät (1) für einen medizinischen Wirkstoff, insbesondere einen Pen-Injektor, soll die Abfüllung auch großer Stückzahlen von Ampullen (2) für solche Injektoren besonders erleichtern. Dazu umfasst der Nadelkopf (6, 6') erfindungsgemäß eine auf einen Anschlussstutzen (50) einer Wirkstoffampulle (2) aufschiebbaren Innenkappe (58), die in einem als Aufprellkappe (66) ausgestalteten Bereich eine Anzahl von Rastelementen (74) umfasst, und eine auf die Innenkappe (58) aufschiebbaren Außenkappe (80), die in auf die Innenkappe (58) aufgeschobenem Zustand die Rastelemente (74) radial fixiert.

## Beschreibung

Die Erfindung bezieht sich auf einen Nadelkopf für ein Injektionsgerät für einen medizinischen Wirkstoff, insbesondere für einen so genannten Pen-Injektor. Sie betrifft weiterhin ein Injektionsgerät mit einem solchen Nadelkopf.

Für Patienten mit chronischen Erkrankungen, denen regelmäßig medizinische Wirkstoffe verabreicht werden müssen, und die insbesondere auch selbst für die Verabreichungen dieser Injektionen in die Lage versetzt werden sollen, werden in großer Anzahl geeignete Injektionsgeräte bereitgestellt. Insbesondere sollen auch mittels eines solchen Injektionsgeräts bevorzugt aus dem Inhalt eines im Injektionsgerät enthaltenen Vorratsbehälters mehrere Injektionen gegeben werden können. Solche Injektionsgeräte werden z.B. von Diabetikern verwendet, um sich regelmäßig Injektionen mit Insulin zu geben. Solche Injektionsgeräte für den Selbstgebrauch werden üblicherweise auch als "Pen-Injektoren" bezeichnet, insbesondere da sie im Hinblick auf die Alltagstauglichkeit üblicherweise in Form und Größe an übliche Schreibstifte erinnern.

Ein solcher Pen-Injektor, oder das Injektionsgerät im allgemeinen, umfasst üblicherweise eine so genannte Ampulle als den eigentlichen Wirkstoffbehälter, in der der Wirkstoff, also beispielsweise das zu verabreichende Insulin, geeignet vorgehalten ist. Eine solche Ampulle wird vorbefüllt bereitgestellt, und für den eigentlichen Gebrauch einerseits an einem zur Anbringung einer Injektionsnadel vorbereiteten Nadelkopf mit der Injektionsnadel versehen und andererseits an ihrem dem Nadelkopf gegenüberliegenden Ende mit einem geeigneten Betätigungselement versehen. Das Betätigungselement wirkt dabei auf einen in der Ampulle verschiebbar angeordneten Stopfen, über den der Wirkstoff der am Nadelkopf angebrachten Injektionsnadel zugeführt und über diese ausgebrachte werden kann.

Gerade bei Diabetes und bei der Verabreichung von Insulin muss die zu injizierende Dosis üblicherweise, meist sogar unmittelbar vor der jeweiligen Injektion, an den aktuellen Blutzuckerspiegel des Patienten angepasst werden. zu diesem Zweck kann das Injektionsgerät zur Abgabe einer einstellbaren Dosis ausgelegt sein, wobei eine solche Einstellung üblicherweise über das entsprechend ausgelegte Betätigungselement erfolgt. Dieses kann beispielsweise eine Kolbenstange mit Außengewinde aufweisen, die im Gehäuse des Injektionsgeräts und somit in der Ampulle axial verschiebbar angeordnet sein kann. Die Dosiseinstellung kann dann beispielsweise durch Verdrehung eines äußeren Gehäuseteils mit Innengewinde erfolgen, durch das die Kolbenstange axial um eine vorgegebene Wegstrecke verschoben wird. Hierdurch ist eine vergleichsweise exakte Dosierung der zu injizierenden Flüssigkeitsmenge möglich.

Solche Injektionsgeräte sind mittlerweile weit verbreitet. Der Wirkstoff wird dabei wie erwähnt für den Anwender in den vorbefüllten Ampullen bereitgestellt. Bis zum Einsatz sollten diese Ampullen somit im Sinne einer guten Lagerfähigkeit und guten Handhabbarkeit sicher und dicht verschlossen sein, um unerwünschte Wirkstoffverluste zu minimieren oder möglichst ganz zu vermeiden. Weiterhin sollen solche Ampullen im Hinblick auf den großen Bedarf auf besonders einfache Weise in großen Stückzahlen herstellbar und auch im industriellen Maßstab befüllbar sein.

Der Erfindung liegt daher die Aufgabe zugrunde, einen insbesondere zur Verwendung mit der Ampulle eines solchen Pen-Injektors vorgesehenen Nadelkopf anzugeben, mit dem die Abfüllung auch großer Stückzahlen solcher Ampullen besonders erleichtert wird. Des Weiteren soll ein Injektionsgerät mit einem solchen Nadelkopf angegeben werden.

Bezüglich des Nadelkopfs wird diese Aufgabe erfindungsgemäß mit einer auf einen Anschlussstutzen einer Wirkstoffampulle aufschiebbaren Innenkappe, die in einem als Aufprellkappe ausgestalteten Bereich eine Anzahl von Rastelementen umfasst, und mit einer auf die Innenkappe aufschiebbaren Außenkappe, die in auf die Innenkappe aufgeschobenem Zustand die Rastelemente radial fixiert.

Die Erfindung geht dabei von der Überlegung aus, dass zur Vereinfachung des Abfüllprozesses insbesondere auch das nach dem eigentlichen Einfüllen des Wirkstoffs in die Ampulle vorgesehen dichte Verschließen der Ampulle besonders einfach und effizient ausgestaltet sein sollte. Zu diesem Zweck sind gemäß einem Aspekt der Erfindung und in als eigenständig erfinderisch angesehener Ausgestaltung der Nadelkopf und daran angepasst die Ampulle für ein neuartiges Abfüllkonzept ausgelegt, bei dem die Einfüllung des Wirkstoffs in die Ampulle über deren distales, den für die Anbringung der Injektionsnadel tragenden Nadelkopf vorgesehenes Ende erfolgen soll. Der Nadelkopf sollte dafür für eine einfache und gut kontrollierbare Anbringung am distalen Ende der Ampulle ausgelegt sein, nachdem diese mit dem Wirkstoff befüllt wurde. Gemäß einem Aspekt der Erfindung ist hierfür der Nadelkopf für die Anbringung an der Ampulle mittels einer Schnapp- oder Rastverbindung ausgestaltet, bei der der Nadelkopf in einer im Wesentlichen linearen Bewegung auf einen entsprechenden Anschlussstutzen der Ampulle aufgesteckt oder aufgeprellt wird. Um dabei aber dennoch einerseits einen zuverlässigen Halt des Nadelkopfs auf der Ampulle und andererseits eine hohe Dichtheit beim Verschließen der Ampulle sicherzustellen, ist der Nadelkopf gemäß einem Aspekt der Erfindung mehrteilig ausgeführt. Insbesondere sollte er als erste Komponente eine Innenkappe aufweisen, die auf vergleichsweise einfache Weise auf den Anschlussstutzen der Ampulle aufsteckbar und mit diesem verrastbar ist und zudem ein geeignetes Dichtelement zum dichtenden Verschließen des distalen Endes der Ampulle aufweist. Zudem sollte er als zweite Komponente ein geeignetes Sicherungsmittel aufweisen, mit dem die auf den Anschlussstutzen aufgesteckte Innenkappe auf einfache Weise in ihrer Position fixiert werden kann.

Vorteilhafte Ausgestaltungen der Erfindung sind Gegenstand der Unteransprüche.

Vorteilhafterweise umfasst die Innenkappe des Nadelkopfs einen Dichtkörper aus einem im Vergleich zur Aufprellkappe weicheren Material, vorzugsweise TPE.

Die Außenkappe des Nadelkopfs ist in ganz besonders vorteilhafter Ausgestaltung an ihrem Außenmantel mit einem Außengewinde versehen, über das insbesondere ein die Injektionsnadel tragender Nadelaufsatz anbringbar ist. Vorzugsweise ist das Außengewinde dabei somit hinsichtlich seiner Gewinde- oder Geometrieparameter an den vorgesehenen Nadelaufsatz, beispielsweise einen Standard-Nadelaufsatz für Pen-Injektoren, angepasst. Im montierten, d. h. vollständig auf den Anschlussstutzen der Ampulle aufgeschobenem Zustand überdeckt dabei der mit dem Außengewinde versehene Bereich des Außenmantels der Außenkappe den als Aufprellkappe mit Rastelementen ausgestalteten Teil der Innenkappe und umfasst diese. Das Außengewinde trägt somit gemäß einem Aspekt der Erfindung in zusätzlich stabilisierender Weise zur radialen Fixierung der Rastelemente der Innenkappe und damit zu deren Fixierung und sicherer Positionierung am Anschlussstutzen der Ampulle bei.

Bezüglich des Injektionsgeräts mit einer Wirkstoffampulle wird die genannte Aufgabe gemäß einem Aspekt der Erfindung gelöst, indem die Wirkstoffampulle an ihrem distalen Ende mit dem Dichtkörper eines Nadelkopfs der vorstehend beschriebenen Art verschlossen ist.

Gemäß einem Aspekt der Erfindung sind die Vorteile der beschriebenen Ausgestaltung des Nadelkopfs gerade für vorbefüllte Wirkstoffampullen, insbesondere zum Verschließen der Wirkstoffampullen nach ihrer Befüllung mit Wirkstoff, besonders gut nutzbar. Vorteilhafterweise ist somit die Wirkstoffampulle des Injektionsgeräts mit dem zu verabreichenden Wirkstoff vorbefüllt.

Weitere vorteilhafte und/oder als eigenständig erfinderisch angesehene Aspekte der Erfindung sind in den Unteransprüchen angegeben und/oder im nachfolgenden Ausführungsbeispiel eingehender erläutert.

Ein Ausführungsbeispiel der Erfindung wird anhand einer Zeichnung näher erläutert. Darin zeigen:
- FIG. 1: ein Injektionsgerät in seitlicher Ansicht,
- FIG. 2: das Injektionsgerät nach FIG. 1 im Längsschnitt,
- FIG. 3: eine Ampulle des Injektionsgeräts nach FIG. 1 in perspektivischer Ansicht in Explosionsdarstellung,
- FIG. 4: eine Ampulle des Injektionsgeräts nach FIG. 1 im Längsschnitt in Explosionsdarstellung,
- FIG. 5: einen in der Ampulle nach FIG. 3 geführten Stopfen im Längsschnitt,
- FIG. 6: den Stopfen nach FIG. 5 in perspektivischer Ansicht,
- FIG. 7: den Stopfen nach FIG. 5 in seitlicher Ansicht,
- FIG. 8: den Stopfen nach FIG. 5 in perspektivischer Ansicht,
- FIG. 9: einen zur Anbringung am distalen Ende der Ampulle nach FIG. 3 vorgesehenen Nadelkopf im Längsschnitt,
- FIG. 10: den Nadelkopf nach FIG. 9 in seitlicher Ansicht,
- FIG. 11: den am distalen Ende der Ampulle gem. FIG. 3, 4 angebrachten Nadelkopf im Längsschnitt,
- FIG. 12: den am distalen Ende der Ampulle gem. FIG. 3, 4 angebrachten Nadelkopf in seitlicher Ansicht,
- FIG. 13: eine alternative Ausführungsform eines zur Anbringung am distalen Ende der Ampulle nach FIG. 3 vorgesehenen Nadelkopfs im Längsschnitt,
- FIG. 14: den Nadelkopf nach FIG. 13 in seitlicher Ansicht,
- FIG. 15: den am distalen Ende der Ampulle gem. FIG. 3, 4 angebrachten alternativen Nadelkopf gem. Fig. 13 im Längsschnitt,
- FIG. 16: den am distalen Ende der Ampulle gem. FIG. 3, 4 angebrachten Nadelkopf gem. Fig. 13 in seitlicher Ansicht,
- FIG. 17: einen Nadelaufsatz zur Anbringung an einem Nadelkopf gem. Fig. 9, 13 im perspektivischen Schnitt,
- FIG. 18: das distale Ende der Ampulle gem. FIG. 3, 4 nach der Entfernung der Siegelplatte,
- FIG. 19: das distale Ende der Ampulle gem. Fig. 3, 4 mit aufgeschraubtem Nadelhalter im Längsschnitt, und
- FIG. 20: eine Sequenz von Schritten bei der Benutzung des Injektionsgeräts gem. Fig. 1, jeweils im Längsschnitt.

Gleiche Teile sind in allen Figuren mit denselben Bezugszeichen versehen.

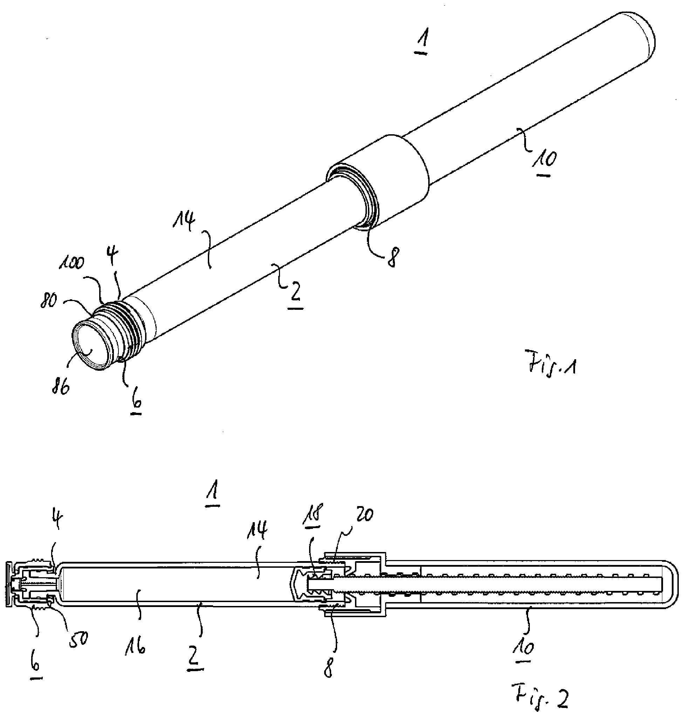

Das Injektionsgerät 1 gemäß FIG. 1 ist als so genannter Pen-Injektor ausgestaltet und zur mehrfachen dosierten Abgabe eines medizinischen Wirkstoffs, insbesondere Insulin oder dergleichen, vorgesehen. Es umfasst im Wesentlichen drei Baugruppen, nämlich eine einen Wirkstoffbehälter bildende Ampulle 2 (auch als Karpule, Kartusche oder Ampullenkörper bezeichnet), die an ihrem distalen Ende 4 einen Nadelkopf 6 trägt und an ihrem proximalen Ende 8 mit einem Betätigungselement 10 verbunden ist. In der Gesamtansicht in FIG. 1 und auch in der Darstellung im Längsschnitt in FIG. 2 ist das Injektionsgerät 1 jeweils mit noch am Nadelkopf 6 nicht angebrachter Injektionsnadel und somit in einem Transport- oder Lagerzustand oder auch in einem verkaufsfertigen Zustand gezeigt. Die Ampulle 2 umfasst entsprechend einer herkömmlichen Bauweise einen zylindrisch oder rohrförmig ein Spritzengehäuse bildenden Hohlkörper 14, der zur Aufnahme des medizinischen Wirkstoffs, beispielsweise des Insulins, vorgesehen ist. In diesem Hohlkörper 14 ist innerhalb des die eigentliche Wirkstoffkammer bildenden Spritzenkanals 16 ein Stopfen 18 geführt, der den Innenraum des Hohlkörpers 14 zu seinem proximalen Ende 8 dichtend abschließt. Zudem ist auch der am distalen Ende 4 des Hohlkörpers 14 angebrachte Nadelkopf 6 im gezeigten Zustand, also noch ohne die Injektionsnadel, derart ausgeführt, dass er den Innenraum des Hohlkörpers 14 dichtend abschließt. Die Ampulle 2 ist somit in ihrer Gesamtheit als abgedichteter Behälter ausgeführt, in dem der medizinische Wirkstoff für eine gewisse Lagerzeit aufbewahrt und vorgehalten werden kann. Insbesondere kann die Ampulle 2 somit als vorbefüllter Wirkstoffcontainer ausgeführt sein.

Hinsichtlich der als eigenständig erfinderisch angesehenen Materialwahl für die Ampulle 2 bzw. den diese bildenden Hohlkörper 14 ist insbesondere hohen Ansprüchen an die zuverlässige vorübergehende Lagerung des Wirkstoffs, insbesondere Insulin, einhergehend mit einer besonders hohen Sicherheit im Umgang mit den Komponenten Rechnung getragen. Der zylindrisch ausgeführte Hohlkörper 14 kann dabei aus COC, PC oder PP gefertigt sein, ist im gezeigten Ausführungsbeispiel aber in als erfinderisch angesehener Ausgestaltung aus dem Hochleistungskunststoff Cyclo-Olefin-Polymer (COP) gefertigt. Dieser Werkstoff zeichnet sich durch hohe Bruchfestigkeit und glasähnliche Transparenz aus. Er setzt zudem keine Alkali-Ionen frei, so dass das Risiko einer pH-Wert-Verschiebung im vorgehaltenen Wirkstoff ausgeschlossen ist.

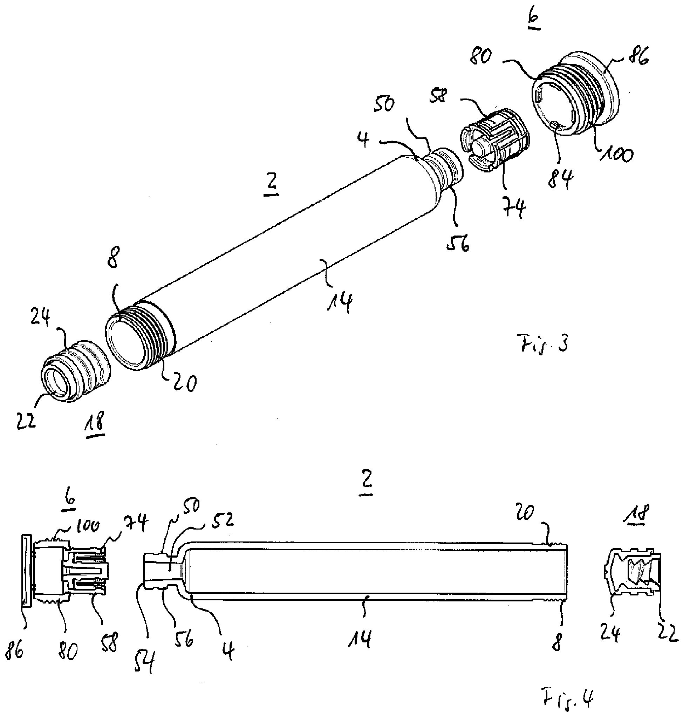

Die als eigenständig erfinderisch angesehene Ampulle 2 ist mit ihren beidseitigen Verschlusselementen, nämlich dem Nadelkopf 6 einerseits und dem Stopfen 18 andererseits, in FIG. 3 in perspektivischer Ansicht und in FIG. 4 im Längsschnitt, jeweils in Explosionsdarstellung, gezeigt. Der Hohlkörper 14 ist dabei an seinem proximalen Ende 8 mit einem Außengewinde 20 versehen, um die Verbindung mit dem Betätigungselement 10 zu ermöglichen. In das proximale Ende 8 ist der Stopfen 18 einbringbar, der im eingebrachten Zustand den Hohlkörper 14 zu seinem proximalen Ende 8 hin dichtend verschließt.

Der Stopfen 18 ist dabei über das am proximalen Ende des Hohlkörpers 14 angeordnete Betätigungselement 10 innerhalb des Hohlkörpers 14 verschiebbar, so dass beispielsweise der im Hohlkörper 14 vorgehaltene Wirkstoff über eine im Nadelkopf 6 gelagerte Hohlnadel ausgetragen und somit appliziert werden kann. Der Stopfen 18 ist dabei derart dimensioniert, dass er dichtend an der Innenwand des Hohlkörpers 14 anliegt; im Längsbereich des Innenraums des Hohlkörpers 14 zwischen dem distalen Ende 4 und dem Stopfen 18 wird somit das Reservoir für den Wirkstoff ausgebildet, in dem dieser vorgehalten werden kann. Bei an den Nadelkopf 6 angebrachter Hohlnadel ragt diese mit ihrem proximalen Ende in dieses Reservoirvolumen hinein, wobei der Stopfen 18 innerhalb des Hohlkörpers 14 in Richtung zum distalen Ende 4 hin verschoben werden kann, so dass der Wirkstoff durch die Hohlnadel aus dem Innenraum herausgedrückt wird. Dies kann insbesondere in einer silikonfrei gehaltenen Ausgestaltung sein.

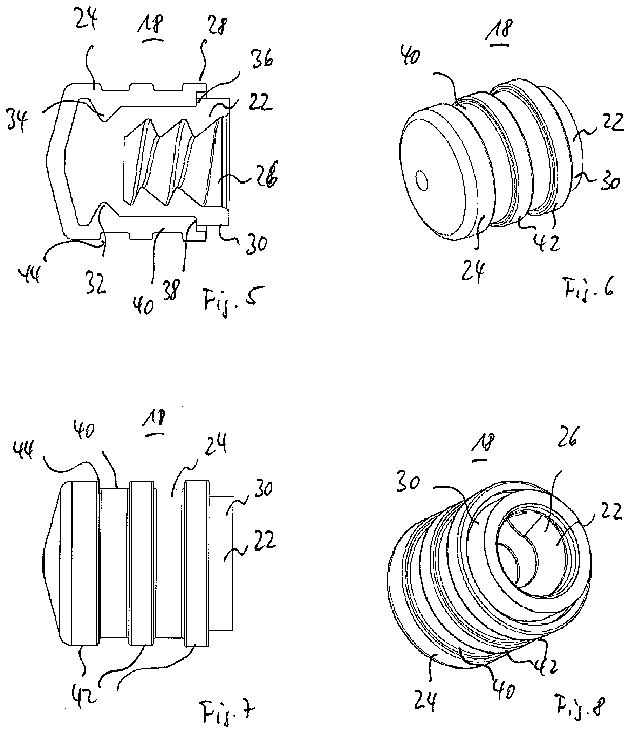

Um auch höchsten Ansprüchen hinsichtlich Dichtheit einerseits und Gleiteigenschaften innerhalb des Hohlkörpers 14 andererseits Genüge zu tun, ist der Stopfen 18 in als eigenständig erfinderisch angesehener Ausgestaltung mehrkomponentig, insbesondere zweikomponentig, ausgeführt. Details zur Ausgestaltung des Stopfens 18 sind den Darstellungen im Längsschnitt in FIG. 5, in perspektivischer Ansicht in FIG. 6, in seitlicher Ansicht in FIG. 7 und in weiterer perspektivischer Ansicht "von hinten" in FIG. 8 entnehmbar.

Der gemäß einem Aspekt der Erfindung zweikomponentig oder als "2K-Stopfen" ausgeführte Stopfen 18 umfasst einen zentralen Stopfenkörper 22 aus einem vergleichsweise harten Kunststoff, im Ausführungsbeispiel und gemäß einem Aspekt der Erfindung aus Polypropylen (PP), der von einem aus im Vergleich dazu weicheren Kunststoffmaterial, im Ausführungsbeispiel und gemäß einem Aspekt der Erfindung TPE, gebildeten Dichtmantel 24 umgeben ist. Der zentrale Stopfenkörper 22 dient dabei im Wesentlichen zur Form- und Strukturstabilität und zur Weiterleitung der eingeleiteten Kräfte, insbesondere in Längsrichtung bei der Verschiebung des Stopfens 18 innerhalb des Hohlkörpers 14. Dazu kann der zentrale Stopfenkörper 22 innenseitig mit einem Aufnahmekanal 26, beispielsweise mit einem Gewinde, versehen sein, über den eine Verbindung mit dem Betätigungselement 10 ermöglicht ist. Durch die als eigenständig erfinderisch angesehene Ausführung des Stopfens 18 als 2K-Stopfen mit einer Kombination des zentralen Stopfenkörpers 22 mit dem vergleichsweise weichen Dichtmantel 24 kann mittels geeigneter Geometrie- und Parameterwahl (beispielsweise genaue Materialzusammensetzung oder Auswahl des verwendeten Kunststoffs) dieser Komponenten einerseits die Anpresskraft des Dichtmantels 24 an die Innenwand des Hohlkörpers 14 und somit die Dichtigkeit des Systems ebenso gut und genau eingestellt werden wie die Gleitreibungs-kräfte beim Verschieben des Stopfens 18 im Hohlkörper 14. Damit kann ein solchermaßen aufgebauter Stopfen 18 gezielt zur Anpassung an die Bedürfnisse bei der Verwendung des Injektionsgeräts 1, beispielsweise bei einer hoch präzise dosierten Abgabe des Wirkstoffs durch den Patienten selbst mit entsprechend feinfühliger Handhabung, angepasst werden.

Des Weiteren kann durch die Kombination dieser Komponenten die üblicherweise bei der Verwendung reiner TPE-Stopfen bei langer Lagerzeit auftretende Schrumpfung und damit ein Dichtigkeitsverlust vermieden werden. Ein solcher 2K-Stopfen ermöglicht somit unter anderem auch zuverlässig vergleichsweise lange Lagerzeiten vorbefüllter Spritzen.

Diese gemäß einem Aspekt der Erfindung vorgesehene Bauweise, also die Bereitstellung des inneren, vergleichsweise härteren Stopfenkörpers 22 mit einer Ummantelung durch den vergleichsweise weicheren Dichtmantel 24, mit dem im Aufnahmekanal 26 vorgesehen stabilen Gewinde wird gerade für die Verwendung in einem als Pen-Injektor ausgestalteten Injektionsgerät 1 als besonders vorteilhaft angesehen. Durch diese Bauweise kann der Stopfenkörper 22 und mit diesem der Stopfen 18 insgesamt fest mit einem Betätigungselement des Injektionsgeräts 1 verbunden werden. Dadurch können axiale Verschiebungen des Stopfens 18 innerhalb des Hohlkörpers 14 sowohl in "Vorwärts- " als auch in "Rückwärtsrichtung" erfolgen, d. h. es können wahl- und bedarfsweise Schub- oder auch Zugwirkungen auf den Stopfen 18 ausgeübt werden.

Der Dichtmantel 24 umgibt den zentralen Stopfenkörper 22 gemäß einem Aspekt der Erfindung in Längsrichtung gesehen nahezu vollständig, wobei lediglich im proximalen Anschlussbereich 28 des Stopfenkörpers 22 ein endseitiger Flansch 30 vom Dichtmantel 24 unbedeckt bleibt. Der zentrale Stopfenkörper 22 ist dabei für eine formschlüssige Fixierung des Dichtmantels 24 mit einer umlaufenden Haltenut 32 versehen, in die eine Innenlippe 34 des Dichtmantels 24 eingreift. Des Weiteren weist der zentrale Stopfenkörper 22 im Bereich des endseitigen Flanschs 30 eine umlaufende Kante 36 auf, auf der eine korrespondierende Innenkante 38 des Dichtmantels 24 aufsitzt.

In einer als eigenständig erfinderisch angesehenen Ausführung ist der Stopfen 18 für eine besonders hohe Dichtwirkung innerhalb des Hohlkörpers 14 ausgelegt. Dabei wird gemäß einem Aspekt der Erfindung gezielt die relativ gute Verformbarkeit des vergleichsweise weichen Dichtmantels 24 mit einer geeigneten Geometriewahl und Formgebung kombiniert. Dazu weist der Dichtmantel 24 einen im Wesentlichen zylindrischen Außenmantel 40 auf, der mit einer Mehrzahl von in Richtung der Zylinderachse gesehen beabstandet zueinander positionierten umlaufenden Dichtrippen 42 versehen ist. Im Bereich der Dichtrippen 42 kann der Stopfen 18 gemäß einem Aspekt der Erfindung mit einem gewissen Übermaß ausgeführt sein, d. h. einen geringfügig größeren Durchmesser aufweisen als der Innendurchmesser des Hohlkörpers 14. Aufgrund der Verformbarkeit des den Dichtmantel 24 bildenden Kunststoffs kann dieser sich somit an die Innenkontur des Hohlkörpers 14 anpassen, wobei aufgrund der Formgebung und der vorgesehenen beabstandet voneinander angeordneten Dichtrippen 42 Ausweichräume bereitgestellt sind, in die die verformten Dichtrippen 42 ausweichen können.

Somit ist es möglich, einerseits auf zuverlässige Weise eine hohe Dichtwirkung zu erzielen, wobei andererseits die auftretenden Haft- und Gleitreibungskräfte infolge des Kontakts mit der Innenwand des jeweiligen Hohlkörpers 14 ausreichend geringgehalten werden können. Gemäß einem Aspekt der Erfindung ist dabei die Breite der oder jeder Dichtrippe 42 geringer gewählt als der Abstand zwischen zwei benachbarten Dichtrippen 42. Durch diese Ausgestaltung des Stopfens 18 kann somit bei hoher Dichtigkeit eine besonders gute und störungsfreie Verschiebbarkeit des Stopfens 18 im Hohlkörper14 erreicht werden.

Zusätzlich begünstigt werden diese Effekte durch die gemäß einem Aspekt der Erfindung vorgesehene Positionierung und Ausgestaltung der Haltenut 32 mit korrespondierender Innenlippe 34, die im Schnitt gesehen jeweils eine Art V-förmige Kontur aufweisen. Damit wird unter Nutzung der Verformbarkeit des relativ weichen Materials des Dichtmantels 24 eine Verformungszone 44 geschaffen, in die hinein Material aus der in Vorschubrichtung des Stopfens 18 gesehen vordersten Dichtrippe 42 ausweichen kann. Damit wird der Reibwiderstand des im Spritzenkörper 2 gleitenden Stopfens 18 gemäß einem Aspekt der Erfindung noch weiter verringert.

Zur weiteren Begünstigung des Reibverhaltens des Stopfens 18 weist gemäß einem Aspekt der Erfindung der Dichtmantel 24 im Bereich des endseitigen Flanschs 30 und seiner Innenkante 38 einen im Vergleich zum Außendurchmesser des zentralen Stopfenkörpers 22 geringfügig vergrößerten Innendurchmesser auf, so dass sich im montierten Zustand endseitig ein Spalt in der Art einer umlaufenden Nut 46 zwischen diesen Komponenten ergibt. Durch diese umlaufende Nut 46 wird eine weitere Verformungszone bereitgestellt, in die bei einer Reibbelastung Material der in Vorschubrichtung des Stopfens 18 gesehen letzten Dichtrippe 42 ausweichen kann.

Gemäß einem Aspekt der Erfindung ist der Stopfen 18 durch ein 2K-Spritzgussverfahren hergestellt.

An seinem distalen Ende 4 ist der Hohlkörper 14, wie den Darstellungen in den FIGs. 3, 4 entnehmbar ist, mit einem Anschlussstutzen 50 zur Anbringung des Nadelkopfs 6 versehen. Der Anschlussstutzen 50 weist gemäß einem als eigenständig erfinderisch angesehenen Aspekt der Erfindung einen Innenkanal 52 auf, der sich vom Innenraum des Hohlkörpers 14 ausgehend in Richtung zu seiner endseitigen Stirnfläche 54 hin kontinuierlich aufweitet; insbesondere ist der Innenkanal 52 dabei im Ausführungsbeispiel mit einer konischen Innenfläche versehen. Gemäß einem als eigenständig erfinderisch angesehenen Aspekt ist eine solche Ausgestaltung des Anschlussbereichs für den Nadelkopf 6 gerade für die für einen Pen-Injektor vorgesehene Ampulle 2, insbesondere in synergistischer Kombination mit dem vorstehend beschriebenen Stopfen 18, besonders vorteilhaft, da durch die Kombination dieser Elemente ein innovatives, besonders effizientes Abfüllkonzept zur Befüllung einer großen Anzahl solcher Ampullen 2, auch im industriellen Maßstab, ermöglicht wird. Bei einem solchen Abfüllkonzept kann vorgesehen sein, die an ihrem proximalen Ende 8 bereits mit dem Stopfen 18 dichtend verschlossene Ampulle 2 aufrecht mit ihrem distalen Ende 4 nach oben ausgerichtet von "oben", also über das distale Ende 4, mit dem Wirkstoff zu befüllen. Die Ausgestaltung des Innenkanals 52 als sich zur Stirnfläche 54 aufweitender Kanal ermöglicht dabei die vorübergehende Anbringung eines korrespondierend geformten Einfüllstutzens, der in den Innenkanal 52 eingesteckt werden kann und aufgrund der Formgebung in diesem sicher geführt ist.

Außenseitig ist der Anschlussstutzen 50 mit einer umlaufenden Außenwulst 56 versehen, die ein Rastmittel für die gemäß einem Aspekt der Erfindung vorgesehene Rast- oder Schnappverbindung für die Anbringung des Nadelkopfs 6 bildet. Der Nadelkopf 6 kann, je nach vorgesehenem Einsatzzweck in einem "Single-Dose" Injektor 1, also für die Abgabe lediglich einer Dosis, oder in einem "Multi-Dose" Injektor 1, also für die Abgabe mehrerer Dosen, ausgelegt sein.

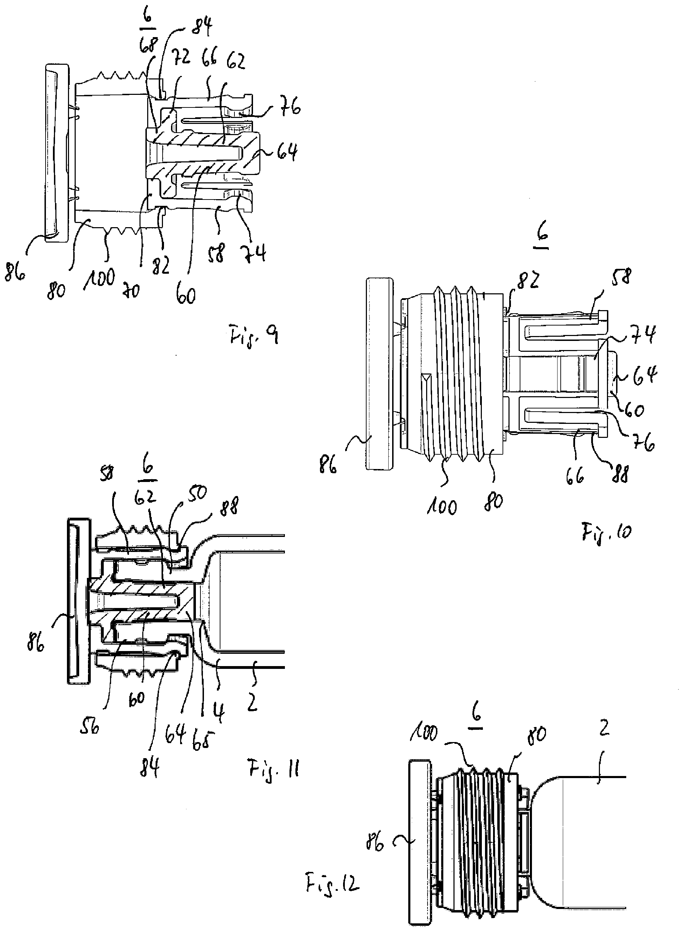

In einer ersten Variante, die insbesondere für eine "Single-Dose"-Anwendung vorgesehen sein kann, und die in FIG. 9 im Längsschnitt und in FIG. 10 in seitlicher Ansicht dargestellt ist, umfasst der Nadelkopf 6 eine Innenkappe 58, die in als eigenständig erfinderisch angesehener Ausgestaltung als zweikomponentiges oder 2K-Bauteil ausgeführt ist. Die Innenkappe 58 umfasst einen zentralen Dichtkörper 60 aus einem vergleichsweise weichen, dichtenden Kunststoff, im Ausführungsbeispiel TPE. Der Dichtkörper 60 ist in seiner Außenkontur in der Art eines Steckzapfens 62 ausgestaltet und an die Innenkontur des Innenkanals 52 angepasst. Er kann somit zunehmend in den Innenkanal 52 eingebracht werden, bis aufgrund der konischen Ausgestaltung des Steckzapfens 62 einerseits und daran angepasst des Innenkanals 52 andererseits ein flächiger und somit besonders dichtender Kontakt zwischen diesen Komponenten entsteht. Damit ist gemäß einem Aspekt der Erfindung eine besonders dichte und formbedingt auch mechanisch besonders stabile Verbindung des Nadelkopfs 6 insgesamt mit dem Hohlkörper 14 erreicht. Endseitig des Steckzapfens 62 weist der Dichtkörper 60 eine in Form eines Septums oder einer durchstechbaren Membran ausgeführte Endplatte 64 auf. Der Steckzapfen 62 ist dabei in als eigenständig erfinderisch angesehener Ausgestaltung im Vergleich zum Innenkanal 52 derart ausgeführt, dass die Endplatte 64 im montierten Zustand nahe der innenseitigen Endöffnung 65 des Innenkanals 52 positioniert ist, in der dieser in den Spritzenkanal 16 innerhalb des Hohlkörpers 14 mündet. Damit soll insbesondere erreicht werden, dass der im Spritzenkanal 16 geführte Stopfen 18 bei vollständiger Bewegung zum distalen Ende 4 der Ampulle 2 hin vollständig oder zumindest nahezu in Kontakt mit der Endplatte 64 kommt, so dass das Totvolumen nach vollständiger Betätigung des Stopfens 18 minimiert oder zumindest besonders klein gehalten werden kann. Die Endplatte 64 ist dazu gemäß einem Aspekt der Erfindung in einer Entfernung von höchstens 25% der Gesamtlänge des Innenkanals 52 von dessen Endöffnung 65 positioniert.

Der Dichtkörper 60 ist gemäß einem Aspekt der Erfindung in einer als zweite Komponente der Innenkappe 58 vorgesehenen Aufprellkappe 66 angeordnet. Die Aufprellkappe 66 umfasst einen eine zentrale Öffnung 68 aufweisenden Ringdeckel 70. Der an sich einstückig ausgeführte Dichtkörper 60 umfasst hingegen an seinem "oberen", der Endplatte 64 gegenüberliegenden Endbereich eine angeformte Außenwulst 72, die mit dem Ringdeckel 70 in Anschlag gebracht ist. Am Ringdeckel 70 der Aufprellkappe 66 ist eine Anzahl von Schnapphaken oder Rastelementen 74 angeordnet, die jeweils mittels einer innenseitig angeformten Rastrippe 76 mit der Außenwulst 56 des Anschlussstutzens 50 in Eingriff bringbar sind. Beim Anbringen der die Aufprellkappe 66 umfassenden Innenkappe 58 am Anschlussstutzen 50 kann diese somit auf den Anschlussstutzen 50 aufgesteckt oder aufgeprellt werden, wobei die Rastelemente 74 zunächst durch die Außenwulst 56 nach au-βen gebogen werden und anschließend, nach weiterem Aufschieben, die Außenwulst 56 hintergreifen und in der Art einer Schnappverbindung mit dieser verrasten. Ein solches Aufprellsystem ist besonders einfach montierbar und damit bevorzugt; alternativ könnte aber auch eine Schraubverbindung der Innenkappe 58 mit dem Anschlussstutzen 50 oder eine andere geeignete Verbindung vorgesehen sein.

Als weitere Komponente, wie dies wiederum aus der Darstellung in Fig. 9, 10 deutlich wird, umfasst der Nadelkopf 6 in einer als eigenständig erfinderisch angesehenen Ausgestaltung eine auf die Aufprellkappe 66 der Innenkappe 58 aufschiebbare Außenkappe 80. Diese kann nach dem Aufprellen der Aufprellkappe 66 und der Verrastung mit der Au-βenwulst 56 von außen umgreifend auf die Aufprellkappe 66 geschoben werden. Damit fixiert sie die Rastelemente 74 radial, so dass diese nicht mehr nach außen ausweichen können. Damit ist die Verrastung der Aufprellkappe 66 mit der Außenwulst 56 nicht mehr ohne weiteres lösbar und somit festgelegt.

Am oberen Rand der Aufprellkappe 66 und im Bereich des Ringdeckels 70 ist die Innenkappe 58 außenseitig mit einer umlaufenden Nut 82 versehen. Somit kann gemäß einem Aspekt der Erfindung in der Art einer Vormontage die Außenkappe 80 auf die Innenkappe 58 aufgesteckt werden, wobei eine Anzahl von innenseitig an der Außenkappe 80 angeordnete Rastnasen 84 in die Nut 82 eingreifen. Der Nadelkopf 6 bildet somit das in FIG. 9 im Längsschnitt und in FIG. 10 in seitlicher Ansicht gezeigte Ensemble, das gemeinsam auf den Hohlkörper 14 aufgesteckt werden kann.

Weiterhin ist gemäß einem Aspekt der Erfindung der Nadelkopf 6 als Originalitätsverschluss ausgeführt. Dazu umfasst er eine in der Art eines Siegels abreißbar an der Au-βenkappe 80 angeformte Siegelplatte 86. Beim erstmaligen Gebrauch des Injektionsgeräts 1 wird die Siegelplatte 86 von der Außenkappe 80 abgerissen, und die darunter liegende Öffnung 68 in der Innenkappe 58 wird zugänglich gemacht. Damit wird für den Benutzer eindeutig erkennbar, dass das Injektionsgerät 1 bereits benutzt und der Wirkstoffcontainer "angebrochen" wurde.

Beim Anbringen des Nadelkopfs 6 kann die Innenkappe 58 somit wie erwähnt auf den Anschlussstutzen 50 des Hohlkörpers 14 aufgeschoben werden, wobei die Rastelemente 74 in der Art einer Schnappverbindung mit der Außenwulst 56 verrasten. Zum endgültigen Anbringen des Nadelkopfs 6 und auch zum Verschließen des distalen Endes 4 der Ampulle 2 kann sodann, wie dies der Darstellung im Längsschnitt gem. FIG. 11 und der Darstellung in seitlicher Ansicht gem. FIG. 12 entnehmbar ist, die Außenkappe 80 noch weiter auf die Innenkappe 58 aufgeschoben werden. Gemäß einem als eigenständig erfinderisch angesehenen Aspekt sind zudem die den unteren oder Schürzenbereich der Aufprallkappe 66 bildenden Rastelemente 74 jeweils mit einer außenseitig angeordneten weiteren Nut 88 versehen, in die die innenseitig an der Außenkappe 80 angeordneten Rastnasen 84 bei vollständig aufgeschobener Außenkappe 80 eingreifen können. Die Außenkappe 80 wird somit gemäß einem Aspekt der Erfindung so weit auf die Innenkappe 58 aufgeschoben, bis die Rastnasen 84 nunmehr in die weitere Nut 88 eingreifen. Damit ist die Außenkappe 80 rastend auf der Innenkappe 58 des Nadelkopfs 6 fixiert, und die Außenkappe 80 umschließt den Schürzenbereich der Aufprellkappe 66 in der Art eines Sicherungsrings. Damit wird die rastende Verbindung der die Außenwulst 56 hintergreifenden Rastelemente 74 radial fixiert, da diese nun nicht mehr nach außen ausweichen können und die Verrastung nunmehr festgelegt ist.

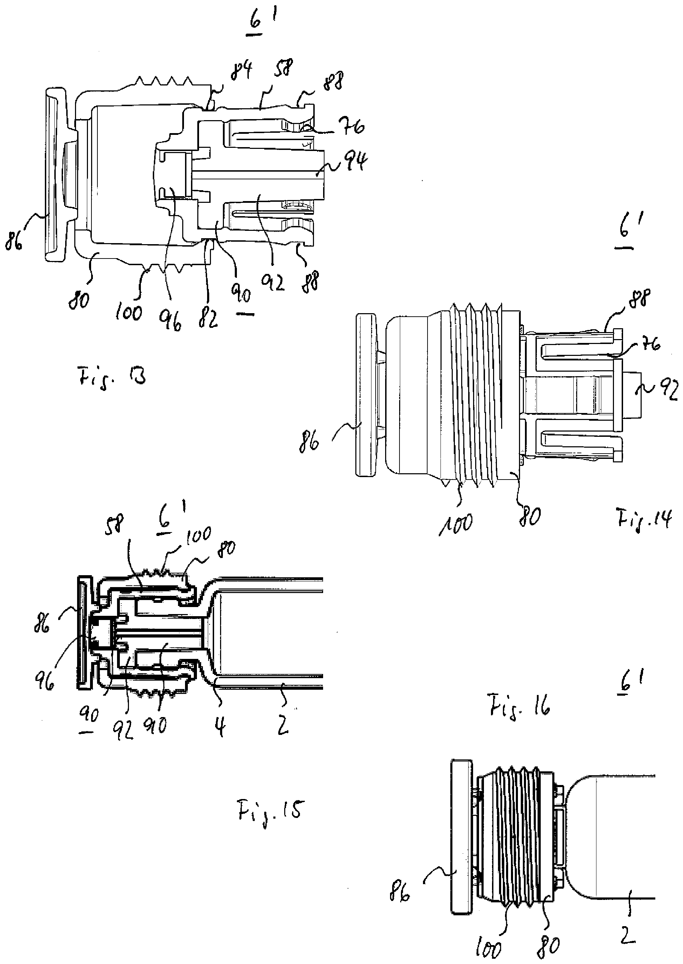

Eine alternative, als eigenständig erfinderisch angesehene Ausführungsform oder Variante des Nadelkopfs 6', die insbesondere für eine "Multi-Dose"-Anwendung oder die mehrfache Abgabe einzelner Wirkstoffdosen aus dem Injektionsgerät 1 vorgesehen sein kann, ist in FIG. 13 im Längsschnitt und in FIG. 14 in seitlicher Ansicht dargestellt. In dieser Ausführungsform ist innerhalb der Innenkappe 58 des Nadelkopfs 6', bei ansonsten im Wesentlichen gleicher Bauweise und Funktionalität der zuvor beschriebenen Komponenten, anstelle des Dichtkörpers 60 ein mehrkomponentiges Kolbenventil 90 angeordnet. Dieses umfasst einerseits einen in seiner Außenkontur ebenfalls, wie vorstehend beschrieben, in der Art eines Steckzapfens ausgestalteten und an die Innenkontur des Innenkanals 52 angepassten Kanalkörper 92. Dieser kann somit, ähnlich wie der zuvor beschriebene Steckzapfen 62, zunehmend in den Innenkanal 52 eingebracht werden, bis aufgrund der konischen Ausgestaltung des Kanalkörpers 92 einerseits und daran angepasst des Innenkanals 52 andererseits ein flächiger und somit besonders dichtender Kontakt zwischen diesen Komponenten entsteht. Damit ist gemäß einem Aspekt der Erfindung auch in dieser Ausführungsform eine besonders dichte und formbedingt auch mechanisch besonders stabile Verbindung des Nadelkopfs 6 insgesamt mit dem Hohlkörper 14 erreicht. Innerhalb des Kanalkörpers 92 ist ein Medienkanal 94 angeordnet, über den Medium vom Innenraum der Ampulle 2 nach Außen gefördert werden kann.

Ergänzend dazu umfasst das Kolbenventil 90 einen Ventilkolben 96. Dieser kann ebenfalls als 2K-Bauteil ausgeführt sein und insbesondere für eine selbsttätige Ventilfunktion ausgestaltet sein, bei der bei einer Druckbeaufschlagung des vorgeschalteten Medienkanals, also bei aus dem Innenraum der Ampulle 2 zugeführtem Medium, eine ausgangsseitig angeordnete Ventilmembran angehoben oder in eine Öffnungsposition gebracht wird, so dass die entsprechenden Ventilkanäle freigelegt werden und das Medium ausströmen kann.

Die Anbringung des Nadelkopfs 6' an der Ampulle 2 durch Aufschnappen oder per Rastverbindung ist ansonsten identisch zu der des vorstehend beschriebenen Nadelkopfs 6. Auch in dieser Ausführungsform wird zum endgültigen Anbringen des Nadelkopfs 6' und auch zum Verschließen des distalen Endes 4 der Ampulle 2, wie dies der Darstellung im Längsschnitt gem. FIG. 15 und der Darstellung in seitlicher Ansicht gem. FIG. 16 entnehmbar ist, die Außenkappe 80 bis zur endgültigen Verrastung und Fixierung auf die Innenkappe 58 aufgeschoben.

Wie den Darstellungen der vorstehend beschriebenen Figuren entnehmbar ist, ist die Au-βenkappe 80 des Nadelkopfs 6, 6' gemäß einem Aspekt der Erfindung an ihrem Außenmantel mit einem Außengewinde 100 versehen. Dieses ist in als eigenständig erfinderisch angesehener Ausgestaltung zur Anbringung eines eine Hohlnadel 102 tragenden Nadelaufsatzes 104, insbesondere eines Standard-Pen-Aufsatzes, vorgesehen ist. Bevorzugt und gemäß einem Aspekt der Erfindung ist dabei des Außengewinde 100 hinsichtlich seiner Geometrie und seiner Gewindeparameter, also beispielsweise Durchmesser und/oder Gewindesteigung, an die entsprechenden Parameter herkömmlicher und standardmäßig verwendeter Pen-Aufsätze angepasst. Zusammenfassend umfasst der als eigenständig erfinderisch angesehene Nadelkopf 6,6' somit im montierten Zustand eine einen Dichtkörper 60 bzw. ein Kolbenventil 90 umfassende Innenkappe 58, die mittels der Aufprellkappe 66 rastend am distalen Ende 4 der Ampulle 2 befestigt ist, wobei eine die Aufprellkappe 66 fixierende Außenkappe 80 außenseitig, also insbesondere in dem die Aufprellkappe 66 umschließenden Mantelbereich, mit einem Außengewinde 100 zur Anbringung des Nadelaufsatzes 104 versehen ist.

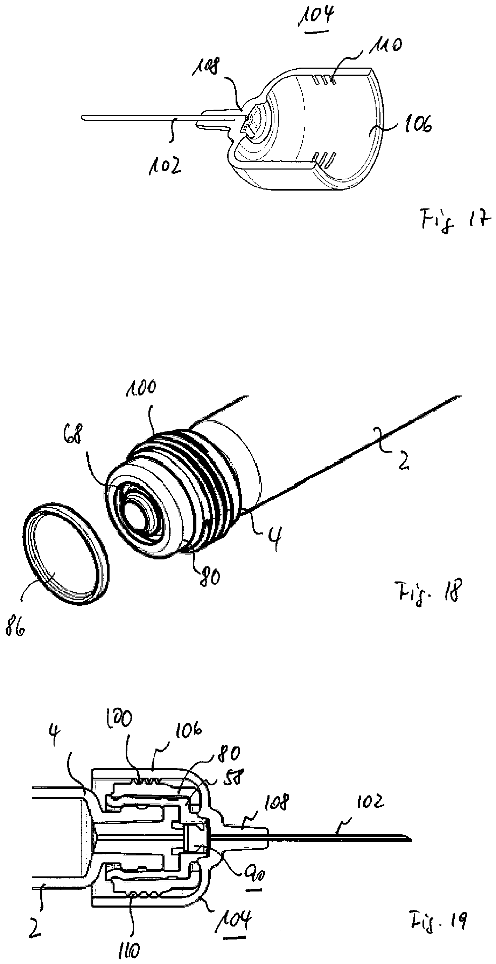

Der Nadelaufsatz 104 ist in Fig. 17 in perspektivischer Schnittansicht gezeigt. Als Grundkörper umfasst er eine Kappe 106, die einen angeformten Nadelhalter 108 umfasst. An ihrem Innenmantel ist die Kappe 106 mit einem Innengewinde 110 versehen, das hinsichtlich seiner Geometrie- und Gewindeparameter an das Außengewinde 100 angepasst und somit auf dieses aufschraubbar ist.

Um das Injektionsgerät 1 einsatzfertig zu machen, wird vom Nadelkopf 6,6' zunächst die Siegelplatte 86 entfernt, so dass das darunter liegende Dichtelement 60 bzw. Kolbenventil 90 freigelegt und damit zugänglich gemacht wird. Dies ist in FIG. 18 anhand des ausschnittsweise gezeigten distalen Endes 4 der Ampulle 2 gezeigt. Anschließend kann dann der Nadelaufsatz 104 aufgeschraubt werden, wie das beispielhaft für den Nadelkopf 6' im Längsschnitt in Fig. 19 gezeigt ist.

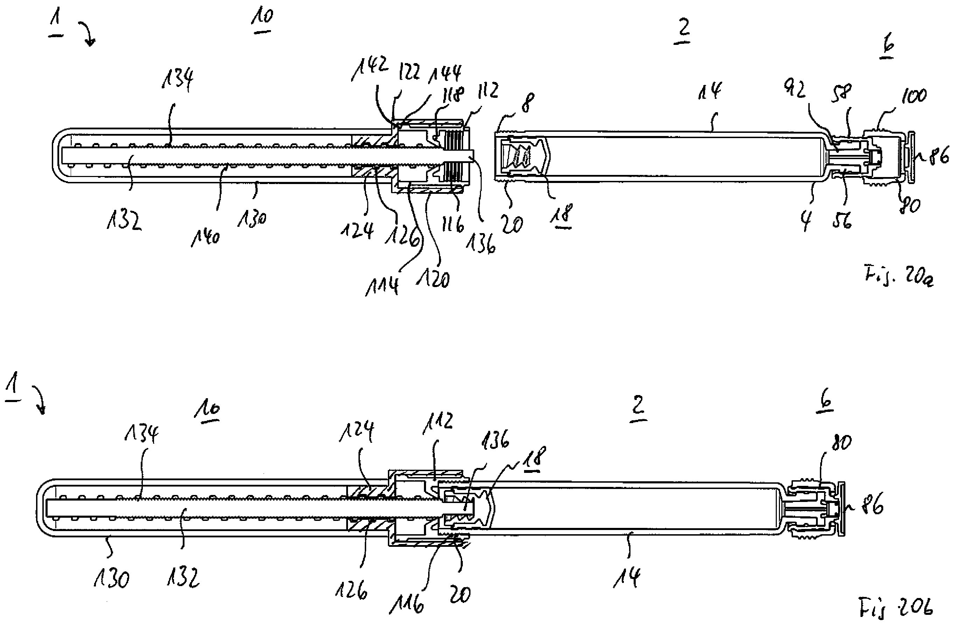

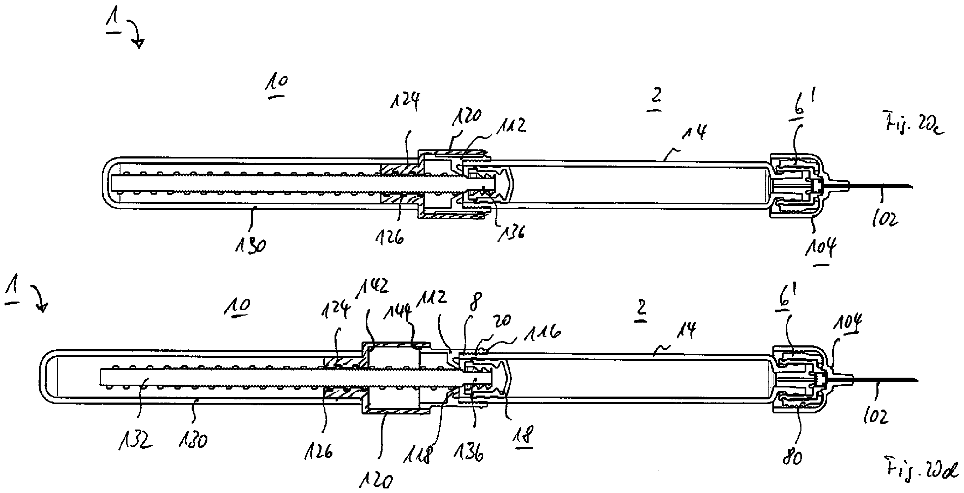

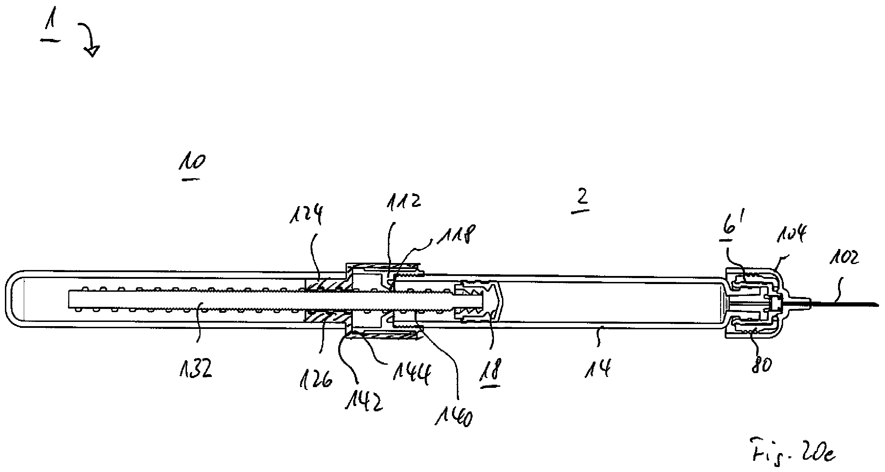

Der Einsatz und die Verwendung des Injektionsgeräts 1, gerade in der Variante als "Multi-Dose"-Gerät bei der Verabreichung einer Wirkstoffdosis, ist in Fig. 20 anhand einer Sequenz von Darstellungen jeweils im Längsschnitt gezeigt. Dabei wird die mit dem Wirkstoff, beispielsweise mit dem Insulin, befüllte und einerseits mit dem Stopfen 18 und andererseits mit dem Nadelkopf 6, 6' verschlossene Ampulle 2 zunächst (Fig. 20a) mit dem Betätigungselement 10 verbunden, indem dieses auf das Außengewinde 20 aufgeschraubt wird. Das Betätigungselement 10 ist seinerseits mehrteilig und in als eigenständig erfinderisch angesehener Ausgestaltung gemäß der nachfolgenden Beschreibung als Betätigungs- und Dosierelement ausgeführt. Es umfasst eine Überwurfmutter 112, die im Wesentlichen von einem hohlen Mantelzylinder 114 gebildet ist. Dieser ist innenseitig mit einem distalen Innengewinde 116 versehen, das in seinen Parametern an das Außengewinde 20 der Ampulle 2 angepasst ist, so dass die Überwurfmutter 112 auf das proximale Ende 8 der Ampulle 2 aufgeschraubt werden kann. Weiterhin ist in etwa mittig im Mantelzylinder 114 eine Anzahl von Rastfedern 118 angeordnet.

Der Mantelzylinder 114 der Überwurfmutter 112 ist in einem Rohrstück 120 einer Drehmutter 122 axial verschiebbar geführt. Die Drehmutter 122 weist einen Spindelkörper 124 mit innenseitig angeordnetem Spindel- oder Steilgewinde 126 auf, und sie ist an einem Außengehäuse 130 angeordnet. Innerhalb des sich länglich erstreckenden, außenseitig einen Griffbereich für den Verwender bildenden Außengehäuses 130 ist als wesentliches Dosiermittel zur Voreinstellung der abzugebenden Wirkstoffmenge eine Kolbenstange 132 angeordnet. Die Kolbenstange 132 ist an ihrer Außenseite mit einem an das Steilgewinde 126 angepassten Steil-Außengewinde 134 versehen und mit diesem durch den Spindelkörper 124 geführt. Endseitig mündet die Kolbenstange 132 in einen in den Stopfen 18 des Injektionsgeräts 1 einsteckbaren Verbindungszapfen 136. Des Weiteren ist die Kolbenstange 132 außenseitig mit einem das Außen-Steilgewinde 134 überlagernden Rast-Feingewinde 140 versehen.

In einem ersten Schritt wird das Betätigungselement 10 am proximalen Ende 8 der Ampulle 2 befestigt, indem das Innengewinde 116 der Überwurfmutter 112 auf das Außengewinde 20 der Ampulle aufgeschraubt wird. Dabei dringt der Verbindungszapfen 136 der Kolbenstange 132 in den Aufnahmekanal 26 des in der Ampulle 2 geführten Stopfens 18 ein und stellt so eine Verbindung von Kolbenstange 132 und Stopfen 18 her. Von der solchermaßen verbundenen, in Fig. 20b gezeigten Einheit wird anschließend die Siegelplatte 86 abgerissen und der Nadelaufsatz 104 aufgeschraubt. In diesem, in Fig. 20c gezeigten Zustand ist das Injektionsgerät 1 nunmehr einsatzfertig.

Zur Einstellung der vorgegebenen Dosis oder Abgabemenge wird dann das Außengehäuse 130 einschließlich des darin befestigten Spindelkörpers 126 um einen vorgegebenen, beispielsweise mittels eines geeignet platzierten Anschlags begrenzten, Verdrehwinkel gegenüber der Ampulle 2 und der daran befestigten Überwurfmutter 112 verdreht. Damit verdreht sich der Spindelkörper 126 ebenfalls gegenüber der drehfest mit der Überwurfmutter 112 verbundenen Kolbenstange 132. Die Verdrehung um einen vorgegebenen Verdrehwinkel wird somit durch den durch die Paarung von Steilgewinde 126 und Steil-Außengewinde 134 gebildeten Spindelantrieb in einen definierten axialen Versatz oder Hub des Außengehäuses 130 gegenüber der fest mit der Ampulle 2 verbundenen Überwurfmutter 112 umgesetzt. Deutlich ist dieser Versatz in der Darstellung gem. Fig. 20d, im Vergleich zum vorherigen Zustand gem. Fig. 20c, erkennbar.

Anschließend kann die Medikamentendosis durch Drücken des Außengehäuses 130 ausgebracht werden. Dabei wird das Außengehäuse 130 und mit diesem, aufgrund der Lagerung der Kolbenstange 132 im Spindelkörper 126, die Kolbenstange 132 und der an deren Ende befestigte Stopfen 18 zum distalen Ende 4 der Ampulle 2 hin bewegt, wodurch der Wirkstoff durch die Hohlnadel 102 ausgebracht wird. Die Ausbringung erfolgt dabei so lange, bis der Spindelkörper 126 mit seiner eine Anschlagsfläche 142 bildenden Unterseite am oberen Rand 144 der Überwurfmutter 112 anschlägt. Damit ist die ausgegebene Wirkstoffmenge auf den durch Gewindeparameter und Verdrehwinkel vorgegebenen Maximalbetrag begrenzt; eine exakte Dosierung wird ermöglicht. Beim Verschieben der Kolbenstange 132 durch die Überwurfmutter 112 hindurch gleitet im Übrigen das Rast-Feingewinde 140 durch die in der Überwurfmutter 112 angeordneten Rastfedern 118, die aufgrund des Rasteffekts eine "Rückwärtsbewegung" der Kolbenstange 132 verhindern. Das injektionsgerät 1 nach der Ausbringung einer solchen Dosis ist in Fig. 20e gezeigt. Deutlich erkennbar ist dabei der relative Versatz der Kolbenstange 132 innerhalb des Au-βengehäuses 130. Das Injektionsgerät 1 ist nun zum Ausbringung der nächsten Wirkstoffdosis bereit. Die Ausbringung kann dabei so oft wiederholt werden, bis der Stopfen 18 im Bereich des distalen Endes 4 der Ampulle 2 an deren dortige Endfläche bzw. die Endplatte 64 des Nadelkopfs 6, 6' angeschlagen ist; die Ampulle 2 ist dann vollständig entleert und die Dosen verbraucht.

### Bezugszeichenliste

- 1: Injektionsgerät
- 2: Ampulle
- 4: distales Ende
- 6: Nadelkopf
- 8: proximales Ende
- 10: Betätigungselement
- 14: Hohlkörper
- 16: Spritzenkanal
- 18: Stopfen
- 20: Außengewinde
- 22: Stopfenkörper
- 24: Dichtmantel
- 26: Aufnahmekanal
- 28: Anschlussbereich
- 30: Flansch
- 32: Haltenut
- 34: Innenlippe
- 36: Kante
- 38: Innenkante
- 40: Außenmantel
- 42: Dichtrippe
- 44: Verformungszone
- 46: Nut
- 50: Anschlussstutzen
- 52: Innenkanal
- 54: Stirnfläche
- 56: Außenwulst
- 58: Innenkappe
- 60: Dichtkörper
- 62: Steckzapfen
- 64: Endplatte
- 65: Endöffnung
- 66: Aufprellkappe
- 68: Öffnung
- 70: Ringdeckel
- 72: Außenwulst
- 74: Rastelement
- 76: Rastrippe
- 80: Außenkappe
- 82: Nut
- 84: Rastnase
- 86: Siegelplatte
- 88: Nut
- 90: Kolbenventil
- 92: Kanalkörper
- 94: Medienkanal
- 96: Ventilkolben
- 100: Außengewinde
- 102: Hohlnadel
- 104: Nadelaufsatz
- 106: Kappe
- 108: Nadelhalter
- 110: Innengewinde
- 112: Überwurfmutter
- 114: Mantelzylinder
- 116: Innengewinde
- 118: Rastfedern
- 120: Rohrstück
- 122: Drehmutter
- 124: Spindelkörper
- 126: Steilgewinde
- 130: Außengehäuse
- 132: Kolbenstange
- 134: Steil-Außengewinde
- 136: Verbindungszapfen
- 140: Rast-Feingewinde
- 142: Anschlagsfläche
- 144: oberer Rand

## Patentansprüche

1. Nadelkopf (6, 6') für ein Injektionsgerät (1) für einen medizinischen Wirkstoff, insbesondere einen Pen-Injektor, mit einer auf einen Anschlussstutzen (50) einer Wirkstoffampulle (2) aufschiebbaren Innenkappe (58), die in einem als Aufprellkappe (66) ausgestalteten Bereich eine Anzahl von Rastelementen (74) umfasst, und mit einer auf die Innenkappe (58) aufschiebbaren Außenkappe (80), die in auf die Innenkappe (58) aufgeschobenem Zustand die Rastelemente (74) radial fixiert.

2. Nadelkopf (6, 6') nach Anspruch 1, dessen Innenkappe (58) einen Dichtkörper (60) aus einem im Vergleich zur Aufprellkappe (66) weicheren Material, vorzugsweise aus TPE, umfasst.

3. Nadelkopf (6,6') nach Anspruch 1 oder 2, dessen Außenkappe (80) an ihrem Außenmantel mit einem Außengewinde (100) versehen ist.

4. Injektionsgerät (1) mit einer Wirkstoffampulle (2), die an ihrem distalen Ende (4) mit dem Dichtkörper (60) eines Nadelkopfs (6, 6') nach einem der Ansprüche 1 bis 3 verschlossen ist.

5. Injektionsgerät nach Anspruch 4, dessen Wirkstoffampulle (2) mit dem zu verabreichenden Wirkstoff vorbefüllt ist.
